# EUROPEAN PATENT APPLICATION

(11) **EP 4 437 955 A1**
(43) Date of publication of application: **02.10.2024**
(21) Application number: 23166020.0
(22) Date of filing: 31.03.2023
(51) Int. Cl.: A61B 5/1455

(54) **SYSTEMS AND METHODS FOR OBTAINING A PHOTOPLETHYSMOGRAPHY SIGNAL**

(71) Applicant: Gabi SmartCare, 1435 Mont-Saint-Guibert (BE)
(72) Inventor: STAQUET, Olivier, 1410 Waterloo (BE)
(74) Representative: Calysta NV

(57) **Abstract**

A system for obtaining a photoplethysmography signal, comprising: a first and second LED source emitting first and second radiation at a first and second wavelength; an intensifier system controlling a first and second intensity; a receptor system generating a first PPG-signal based on the first radiation and a second PPG-signal based on the second radiation; an amplifier amplifying the first PPG-signal with a first gain value and the second PPG-signal with a second gain value; a control unit individually controlling: the first intensity and first gain value such that the amplified first PPG-signal is within a first signal range; the second intensity and second gain value such that the amplified second PPG-signal is within a second signal range. A system for determining a skin pattern of the tissue of the patient based on calibration data, and determine a biometric of the patient, taking into account the skin pattern.

## Description

The present invention relates to the field of photoplethysmography (PPG) signals, for example used to determine one or more biometrics of a patient. The invention can e.g. be applied to a wearable device having a system to emit and/or capture a PPG-signal.

It is known that PPG-signals can be used to determine several biometrics of a patient, such as heart rate and oxygen saturation (SpO2). Radiation is usually emitted from one or more light emitting diodes (LEDs) onto tissue of the patient. The radiation reflected by the tissue or the radiation travelling through the tissue, is received by a receptor. The signal received by the receptor is generally referred to as the PPG-signal. Several characteristics of the PPG-signal depend on contents of the tissue, for example the blood in blood vessels. Based on this, the biometrics can be determined.

Several systems and devices are known to determine biometrics using a PPG-signal. A disadvantage of the known systems, is that they do not always produce reliable results for all biometrics and for all patients.

It is an object of the invention to overcome the disadvantages of the prior art, or at least provide an alternative to the prior art. It is in particular an object of the invention to enable reliably determining biometrics for a wide range of patients.

This object is achieved with a system for obtaining a photoplethysmography signal, comprising:
- a first LED source configured to emit first radiation at a first wavelength onto tissue of the patient;
- a second LED source configured to emit second radiation at a second wavelength onto the tissue of the patient;
- an intensifier system configured to control:
   - the first LED source to emit the first radiation with a first intensity, and
   - the second LED source to emit the second radiation a second intensity;
- a receptor system configured to
   - receive at least a part of the first radiation and generate a first PPG-signal based on the first radiation; and
   - receive at least a part of the second radiation and generate a second PPG-signal based on the second radiation;
- an amplifier system configured to
   - receive the first PPG-signal from the receptor system, and amplify the first PPG-signal with a first gain value into an amplified first PPG-signal; and
   - receive the second PPG-signal from the receptor system, amplify the second PPG-signal with a second gain value into an amplified second PPG-signal;
- a control unit configured to individually control:
   - the first intensity and first gain value such that the amplified first PPG-signal is within first signal range;
   - the second intensity and second gain value such that the amplified second PPG-signal is within second signal range.

The invention thus relates to a system for obtaining a photoplethysmography signal, e.g. for determining a biometric of a patient. The system may be a system for obtaining one or more photoplethysmography signals. The biometric may e.g. be a pulse rate, a respiratory rate, or a peripheral oxygen saturation (SpO₂) level. The biometric may e.g. be a biometric determined based on two PPG-signals, e.g. the SpO₂ level. The system can e.g. be part of a wearable device, e.g. being configured to be worn on a limb, e.g. on an arm or leg. The system can e.g. be part of a watch, e.g. a smartwatch or sports watch. The patient can e.g. be a young child, e.g. a neonate or infant or child up to and including 5 or 12 years of age. A neonate is a new-born child, e.g. 28 days of age oryounger. An infant is a young child, e.g. 2 years or age of younger. However, it is envisaged that the invention can also be applied to adults or other patients.

The system comprises a first radiation source, for example a first LED source. The system may e.g. comprise a second radiation source, e.g. a second LED source. The first and/or second radiation source may thus each comprise one or more LEDs. When mentioned herein, LED is used as acronym for Light Emitting Diode. An LED is configured to emit radiation when being subjected to an electric current. The radiation is electromagnetic radiation, generally light, which can be in the visible spectrum and/or in the non-visible spectrum. Although the term LED source is used in singular term, it is envisaged that in practice a single radiation source may comprise a plurality of LEDs, e.g. being grouped and configured to emit radiation at the wavelength.

The first radiation source is configured to emit first radiation at a first wavelength onto tissue of the patient, and the second radiation source configured to emit second radiation at a second wavelength onto the tissue of the patient. Optionally, the first wavelength is different from the second wavelength. Optionally, the first wavelength is within a frequency range of a first colour, and the second wavelength is within a frequency range of a second colour, wherein the first and second colour or different from each other. The first and/or second colour can e.g. include green (e.g. at a wavelength of approximately 526 nm), red (e.g. at a wavelength of approximately 660 nm), infrared (e.g. at a wavelength of approximately 950 nm), or purple. As it is explained in more detail further below, the invention can also be used for systems having more than two LED sources emitting radiation at more than two different wavelengths. Optionally, the tissue on which the first radiation source emits the first radiation is (approximately) the same tissue on which the second radiation source emits the second radiation.

It is envisaged that the system is generally used on living patients, usually humans, in a non-invasive manner. The first and second radiation will therefore in those cases be emitted onto skin of the patient. The first and second radiation will (partially) penetrate into and through the tissue. The tissue may, besides the skin, also e.g. include membranes, muscles, blood vessels, blood, and tendons.

The system further comprises an intensifier system. The intensifier system allows to control the intensity with which radiation is emitted. The first radiation is emitted with a first intensity, and the second radiation with a second intensity. In embodiments wherein the first and/or second radiation source comprise one or more LEDs, the respective intensity can e.g. be controlled by controlling the electrical current provided to the respective LED(s).

The system further comprises a receptor system, configured to receive at least a part of the first radiation and a part of the second radiation. Said part can e.g. be reflected by the tissue of the patient, or have propagated through the tissue of the patient. The receptor system can e.g. comprise one or more receptors, e.g. one or more photoreceptors. For example, in some embodiments, the system can comprise a first (photo)receptor configured to receive first radiation at the first wavelength, and a second (photo)receptor configured to receive second radiation at the second wavelength. It is also possible that the system comprises one or more (e.g. broadband) (photo)receptors configured to receive radiation at at least the first and second wavelength. Combinations of broadband (photo)receptor(s) and (photo)receptors for the first and/or second wavelength are also possible.

The receptor system converts the received radiation into (electrical) signals. In particular, a first PPG-signal is generated based on the first radiation, and a second PPG-signal is generated based on the second radiation. The first and second PPG-signal each are a so-called pleth, which can be used for photoplethysmography.

The quality of the first and second PPG-signal includes the amplitude/strength of the respective signal, as well as the signal to noise ratio, and in some cases the absence of noise. Depending on the biometric to be determined, either of both may be more important. Generally speaking, however, the quality of the first and second PPG-signal depends on an several factors. Firstly, it depends on the first and second intensity, respectively, with which it was emitted by the respective radiation source. Being emitted onto the tissue of the patient, the quality also depends on characteristics of said tissue. Blood flowing through blood vessels are a changing factor in the tissue. The changes caused by the changes of the blood (e.g. pressure, oxygen saturation, ...) can be used to derive biometrics (e.g. pulse rate, SpO2 level, ...) of the patient.

In addition, it has been found that the quality of the respective PPG-signal also depends on patient-specific characteristics. For example, the skin has an influence on how much radiation is reflected, absorbed, or able to travel through the skin. Characteristics such as skin tone, melatonin level, skin thickness, tattoos, scars, skin temperature, have been found to play a role.

The first and second PPG-signal are transmitted as electrical signals, e.g. to a control unit, which allows e.g. determining the biometrics. To allow for satisfactory further processing of these electrical signals, said signals should be strong enough to analyse while not causing any saturation. At the same time, the signal should preferably stay within the operating range of the amplifier. To achieve this, said signals should be within a signal range. The PPG-signals are, therefore, amplified with a respective gain value. In particular, an amplifier system receives the first and second PPG-signal from the receptor system. The amplifier is configured to amplify the first PPG-signal with a first gain value into an amplified first PPG-signal; and to amplify the second PPG-signal with a second gain value into an amplified second PPG-signal.

The system further comprises a control unit. The control unit is configured to control the first intensity and first gain value such that the amplified first PPG-signal is within first signal range; and to control the second intensity and second gain value such that the amplified second PPG-signal is within second signal range. Moreover, the control unit is configured to individually control the first intensity and the first gain value on the one hand, and the second intensity and second gain value on the other hand. Thus, the control unit is able to control the first intensity to be different than the second intensity, and/or control the first gain value to be different than the second gain value. Optionally, the control unit is configured to control each of the first intensity, first gain value, second intensity, and second gain value individually.

The individual control as explained above thus allows to control the quality of the amplified first PPG-signal on the one hand, and the quality of the amplified second PPG-signal on the other hand. The amplified first PPG-signal can be controlled by means of the first intensity and/or the first gain value. The amplified second PPG-signal can be controlled by means of the second intensity and/or the second gain value.

Said individual control advantageously improves the amplified PPG-signals. The inventors have found that the effect of the tissue (in particular the skin) of the patient on the PPG-signal depends on the wavelength of the radiation. Some types of skin have greater reflection at some wavelengths, while other types of skin have greater reflection at other wavelengths. The same holds true for absorptions. When the same control would be applied to the first and the second wavelength, it could occur that the amplified PPG-signal is satisfactory for the first wavelength but not for the second wavelength. These embodiments overcome these problems, which allows to obtain better signals and ultimately determine the biometrics with more accuracy.

The control unit can e.g. be configured to receive the amplified first PPG-signal and/or the amplified second PPG-signal. The control unit may e.g. comprise a memory for storing (historic) data, data based on received amplified PPG-signals, and/or executable instructions. The executable instructions may e.g. be non-transitory computer-readable instructions. The control unit may e.g. comprise a processing unit for processing the received amplified PPG-signals and/or executable instructions, e.g. for obtaining a photoplethysmography signal, e.g. for determining one or more biometrics of the patient. The control unit may e.g. comprise a CPU. The control unit may e.g. comprise a RAM-memory. The control unit may e.g. comprise a flash-memory. The control unit may e.g. comprise or control a communication module, e.g. for communicating with an external device.

In embodiments, the system, e.g. the control unit or an external control unit, is further configured to determine at least one biometric of the patient based on both the amplified first PPG-signal and the amplified second PPG-signal. Thus, both of the first and second PPG-signal are used in the determination of the biometric. The system does not select one of both PPG-signals and dismisses the other. The biometric can e.g. be a peripheral oxygen saturation (SpO2) level, wherein e.g. the first wavelength is in the red-light spectrum and the second wavelength is in the infrared-light spectrum. The biometric can be determined according to any of the known ways for determining the respective biometric. It will be understood, however, that the system can still be configured to determine one or more (other) biometrics on one of the first and second PPG-signal, e.g. pulse rate or respiratory rate.

In embodiments, the control unit is configured to control the first intensity to be different from the second intensity. In embodiments, the control unit is configured to control the first gain value to be different from the second gain value. These embodiments can be combined, such that the control unit is configured to control the first intensity to be different from the second intensity, and the first gain value to be different from the second gain value.

These embodiments are advantageously enabled by the individual control of the first intensity and first gain value on the one hand, and the second intensity and the second gain value on the other hand. As explained above, radiation at different wavelengths may be affected differently by the tissue of the patient. It can, therefore, be advantageous to control the respective intensities and/or gain values to be different from each other. This allows the optimize each amplified PPG-signal individually.

In embodiments, the control unit is configured to individually control: the first intensity within a first operational intensity range and/or first gain value within first operational gain range; and the second intensity within a second operational intensity range and/or second gain value within second operational gain range. Each respective range can e.g. be defined between a respective upper limit and a respective lower limit.

In embodiments, the first operational intensity range is different from the second operational intensity range, and/or the first operational gain range is different from the second operational gain range. Thus, it may be possible that the first LED source can be controlled to emit the first radiation at intensities that are not within the second operational intensity range for the second LED source, and/or vice versa. Similarly, it may be possible that the first PPG-signal can be amplified at gain values that are not within the second operational gain range for the second PPG-signal. These embodiments advantageously take into account that the reflection and transmission of radiation depends on the wavelength. For some wavelengths, certain intensities or gain values will not be useable. By using adapted ranges, the optimal combination of intensity and gain value can be reached quicker. For example, radiation in the green light spectrum may travel easier the tissue than radiation in the red light spectrum.

In embodiments, the control unit is configured to perform a calibration of the system, wherein during the calibration the control unit is configured to, for calibrating the first LED source:
- control the first LED source and the intensifier system to start emitting the first radiation at a starting intensity which is an upper limit of the first operational intensity range,
- control the amplifier system to amplify the first PPG-signal with a starting gain value which is an upper limit of the first operational gain range, and
- assess the amplified first PPG-signal, and if the amplified first PPG-signal exceeds the first signal range:
   - control the intensifier system to lower the first intensity until
      - the amplified first PPG-signal is in the first signal range, or
      - the first intensity reaches a lower limit of the first operational intensity range, and in that case:
         a. control the amplifier system to lower the first gain value;
         b. control the intensifier system increase the first intensity to the upper limit of the first operational intensity range;
- repeat the previous step until the amplified first PPG-signal is within the first signal range.

These embodiments thus relate to a calibration of the system. The calibration is e.g. done to ensure that the first amplified PPG-signal is within the first signal range. The control unit can e.g. be configured to perform the calibration at least each time the system is started, optionally after detecting that the system is used on a patient (e.g. in case of a wearable device detecting that the device is worn by a patient). The calibration can thus e.g. be a starting calibration.

The calibration in these embodiments starts with a starting intensity and a starting gain value, which are both at the upper limits of the respective operational ranges. Thus, the first intensity is the starting intensity and the first gain value is the starting gain value. This may be advantageous, because it is preferred that the quality of the amplified first PPG-signal is as large as possible within the first signal range. This may improve the determination of the biometric. If the amplified first PPG-signal is within the first signal range when the starting intensity and starting gain are being used, the calibration for the first LED source can stop. If on the other hand the amplified first PPG-signal exceeds the first signal range, then at first the first intensity is controlled to be lowered. This is done until the amplified first PPG-signal arrives within the first signal range, or a lower limit of the first operational intensity range is reached. In the first case the calibration for the first LED source can be stopped. In the latter case, the first gain value is lowered and the first intensity is set back to the upper limit of the first operational intensity range. This is then repeated until the amplified first PPG-signal reaches the first signal range.

By individually and sequentially controlling the first intensity and the first gain value, it is ensured that all possible combinations can be included, while gradually lowering the quality of the amplified first PPG-signal.

In embodiments, the control unit is further configured to, for calibrating the second LED source:
- control the second LED source and the intensifier system to start emitting the second radiation at a starting intensity which is an upper limit of the second operational intensity range,
- control the amplifier system to amplify the second PPG-signal with a starting gain value which is an upper limit of the second operational gain range, and
- assess the amplified second PPG-signal, and if the amplified second PPG-signal exceeds the second signal range:
   - control the intensifier system to lower the second intensity until
      - the amplified PPG-signal is in the second signal range, or
      - the second intensity reaches a lower limit of the second operational intensity range, and in that case:
         a. control the amplifier system to lower the second gain value;
         b. control the intensifier system increase the second intensity to the upper limit of the second operational intensity range;
- repeat the previous step until the amplified second PPG-signal is within the second signal range.

In these embodiments, the second LED source is calibrated similarly as explained above with respect to the first LED source, and similar advantages can thus be achieved. Optionally, the control unit is configured to first calibrate the first LED source, and thereafter calibrate the second LED source.

In other embodiments, the control unit is configured to start from lower limits of the first operational intensity range and the first operational gain range. For example, in embodiments, the control unit is configured to perform a calibration of the system, wherein during the calibration the control unit is configured to, for calibrating the first LED source:
- control the first LED source and the intensifier system to start emitting the first radiation at a starting intensity which is a lower limit of the first operational intensity range,
- control the amplifier system to amplify the first PPG-signal with a starting gain value which is a lower limit of the first operational gain range, and
- assess the amplified first PPG-signal, and if the amplified first PPG-signal is below the first signal range:
   - control the intensifier system to increase the first intensity until
      - the amplified first PPG-signal is in the first signal range, or
      - the first intensity reaches an upper limit of the first operational intensity range, and in that case:
         a. control the amplifier system to increase the first gain value;
         b. control the intensifier system lower the first intensity to the lower limit of the first operational intensity range;
- repeat the previous step until the amplified first PPG-signal is within the first signal range.

For example, in embodiments, the control unit is configured to perform a calibration of the system, wherein during the calibration the control unit is configured to, for calibrating the second LED source:
- control the second LED source and the intensifier system to start emitting the second radiation at a starting intensity which is a lower limit of the second operational intensity range,
- control the amplifier system to amplify the second PPG-signal with a starting gain value which is a lower limit of the second operational gain range, and
- assess the amplified second PPG-signal, and if the amplified second PPG-signal is below the second signal range:
   - control the intensifier system to increase the second intensity until
      - the amplified second PPG-signal is in the second signal range, or
      - the second intensity reaches an upper limit of the second operational intensity range, and in that case:
         a. control the amplifier system to increase the second gain value;
         b. control the intensifier system lower the second intensity to the lower limit of the first operational intensity range;
- repeat the previous step until the amplified second PPG-signal is within the second signal range.

In embodiments, the control unit is further configured perform a recalibration during use. During the recalibration, the control unit can e.g. be configured to check whether each amplified PPG-signal is still within the respective signal range, and adapt the respective intensity and/or gain value accordingly. Even if a calibration has been done when the system is started, it may still be advantageous the perform recalibration during use. This is because the effect of the tissue of the patient on the radiation may change during use. For example, as the skin becomes warmer (e.g. from the radiation itself), the reflection absorption of radiation can change. When the skin becomes moistly or wet (e.g. because the patient is sweating), this will also affect the reflection and absorption of radiation. It is also possible that the location at which the radiation interacts with the tissue changes during use, e.g. when the system is moved relative to the tissue.

In embodiments, the control unit is configured to perform the recalibration periodically during use, e.g. after waiting a stabilisation time, e.g. every 50-300 ms, e.g. every 80-200, e.g. every 90-150 ms, e.g. every 100 ms. Periodic recalibration may be advantageous to ensure that amplified PPG-signals remain within the respective signal ranges. It may also be useful to check whether a previous (re)calibration has successfully caused a respective amplified PPG-signals to be within the respective signal range. However, the system, in particular the intensifier system and/or the amplifier system, may require some stabilisation time before the changes to the first intensity and/or first gain value stabilize. The stabilisation time may depend on the hardware.

In embodiments, during the recalibration the control unit is configured to assess the amplified first PPG-signal, and if the amplified first PPG-signal is not within the first signal range, control the first intensity and/or the first gain value to be decreased or increased. In embodiments, during the recalibration the control unit is configured to assess the amplified second PPG-signal, and if the amplified second PPG-signal is not within the second signal range, control the second intensity and/or the second gain value to be decreased or increased. Optionally, the control unit is configured to first control the first or second intensity, respectively, to be decreased or increased, and thereafter control the first or second gain value, respectively, to be decreased or increased. Changing the intensity may have a smaller impact.

In embodiments, the system further comprises a third LED source configured to emit third radiation at a third wavelength onto tissue of the patient. For example, the third wavelength may be within the red-light spectrum, the infrared-light spectrum, the purple-light spectrum, or the green-light spectrum. For example, one of the first, second, or third wavelength may be within the red-light spectrum, another of the first, second, or third wavelength may be within the infrared-light spectrum, and the last of the first, second, or third wavelength may be within the green-light spectrum. All of the features and embodiments mentioned herein with respect to the first and/or second LED source/radiation/wavelength, may similarly be applied, mutatis mutandis, to the third LED source/radiation/wavelength.

In embodiments, the first and/or second LED source and receptor system are arranged next to each other. The receptor system is configured to receive radiation reflected back from the tissue, rather than radiation that penetrated through the tissue.

In embodiments, the system further comprises a fourth LED source configured to emit fourth radiation at a fourth wavelength onto tissue of the patient. Optionally, the fourth wavelength is equal to one of the first, second, or third wavelength. For example, the fourth LED source and one of the first, second, third LED source may both be configured to emit radiation in the green-light spectrum. The wavelengths corresponding with green-light are able to penetrate the skin relatively easily, thereby enabling the detection of a pulse rate. Based on variations in the received radiation, it may e.g. be possible to determine the respiratory rate. By providing two LEDs emitting green-light, the detection and acquisition of the signal are more robust.

In embodiments, the control unit is configured to control the first LED source and the second LED source - and when present the third and/or fourth LED source - , to emit the respective radiation sequentially. For example, first the first radiation is emitted, and thereafter the second radiation is emitted. This is then repeated. When less or more LED sources are present, this can be adapted accordingly.

In embodiments, the control unit is configured to determine a skin pattern of the tissue of the patient based on calibration data, wherein said calibration data e.g. comprises on one or more of the first intensity, the first gain value, the second intensity, and the second gain value. When the third LED source is present, on the calibration data may include the third intensity, and the third gain value.

For each of the wavelengths, the respective intensity and gain value that result in the respective amplified PPG-signal being within the respective signal range, depend on the characteristics of the tissue, e.g. of the skin. Said respective intensity and gain value may e.g. be selected during the calibration and thus be part of calibration data. The different types of tissues that exist, can be divided in a predetermined number of skin patterns, for example seven skin patterns. Based on the calibration data, the control unit can then determine to which skin pattern the respective tissue of the respective patient belongs.

There will usually be a very large number (thousands, tens of thousands, or even millions) of possible settings from the calibration data, when combining all possibilities for the intensities and gain values for each wavelength. However, the possible settings can be clustered in groups, wherein each group represents a skin pattern. The classification of the groups and skin patterns can e.g. be done by doing measurements on test subjects having different skin patterns.

In embodiments, the control unit may further be configured to determine a biometric of the patient, taking into account on the skin pattern. The type of tissue, as identified by the skin pattern, may affect the PPG-signals. Taking this into account when determining the biometric(s) based on said PPG-signals, can allow for more accurate determination of said biometric(s).

For example, the control unit can be configured to determine an SpO2 level as biometric, wherein the control unit is configured to determine a ratio of ratios, and convert the ratio of ratios to an SpO2 level using a transfer function. The control unit can be configured to determine or select the transfer function based on the skin pattern.

The ratio of ratios or the R-value is a well-known determination used for determining an SpO2 level, as is known to the skilled person. Conventionally, the transfer function (sometimes also referred to as the calibration function) is the same for all patients, and can be a compromise to be relatively well-fitting for all skin patterns. The inventors have found that it may be more advantageous to determine different transfer functions depending on the skin pattern, and select the transfer function accordingly. As such, the determination of the SpO2 level can be done more accurately.

It will be understood that the principle of the determination of a skin pattern can also be done with other types of calibration. Therefore, the invention can also relate to :
A system for determining a biometric of a patient based on one or more PPG-signals, comprising a control unit configured to:
- determine or receive calibration data, wherein said calibration data is determined during a calibration of the system, wherein optionally said calibration includes controlling:
   ∘ one or more LED sources for emitting radiation and/or
   ∘ a receptor system and/or an amplifier system for generating said one or more PPG-signals based on received radiation;
- determine a skin pattern of the tissue of the patient based on the calibration data;
- determine a biometric (e.g. an SpO2 level) of the patient, taking into account on the skin pattern.

It will be understood that features explained herein with reference to the system(s)/device(s) described herein have the same meaning with respect to the system for determining a biometric unless explicitly defined otherwise. Features explained with reference to the system(s)/device(s) described herein can be applied mutatis mutandis to the system for determining a biometric to achieve the similar advantages, and vice versa.

The invention further relates to a wearable device for obtaining a photoplethysmography signal, e.g. for determining a biometric of a patient, comprising the system according to any of the embodiments described herein. Wearable means that the patient can wear the device on his body. The wearable device may e.g. comprise a housing, wherein the housing comprises the first and second LED source (and the third when present), the receptor system, the amplifier system, and the control unit. The wearable device may further comprise a band configured to be attached to the housing and to attach the housing to the tissue of the patient.

In embodiments, the wearable device is configured to be worn on an arm. The wearable device is e.g. configured to be arranged on an upper arm.

In embodiments, the wearable device is configured to be worn by e.g. by a young child, e.g. a neonate or infant or child up to and including 5 or 12 years of age. A neonate is a new-born child, e.g. 28 days of age or younger. An infant is a young child, e.g. 2 years or age of younger.

In embodiments, the wearable device comprises a communication module for communicating wirelessly with an external device. The external device may e.g. be user equipment device, such as a tablet, smartphone, or computer. The communication module may e.g. be configured to communicate using a short-range communication method, such as Bluetooth or Wi-Fi. The wearable device may e.g. be configured to communicate raw and/or processed data based on measurement of the at least one sensor via the communication module.

The invention further relates to a system for determining at least one biometric of a patient, comprising the wearable device according to one or more of the embodiments described herein, and a local user equipment device, wherein the user equipment device is configured to wireless communicate with the wearable device for receiving raw and/or processed data based the first and/or second amplified PPG-signal. The local user equipment device may be configured to process raw/and or processed data, and/or to visualize the data received from the control unit. The local user equipment device may e.g. comprise a processing unit, memory, and/or screen. The local user device may e.g. be a smartphone or tablet.

In embodiments, the system further comprises a cloud-based infrastructure configured to receive raw and/or processed data from the local user equipment device, and comprising at least one server having a processing unit for further processing said raw and/or processed data. The cloud-based infrastructure may e.g. be configured to communicate with the local user equipment device via an internet communication, e.g. WI-FI, 3G, 4G, or 5G.

In embodiments, the system further comprises practitioner portal, configured to receive processed data from the cloud-based infrastructure, and configured to visualize data to a practitioner. Based on the visualized data, the practitioner can determine the health of the patient and propose the suitable medical treatment when required. The practitioner portal may e.g. be a web portal accessible via an internet browser, or an app portal accessible via an application or executable program on a user equipment device.

In embodiments, the system further comprising a caregiver portal, configured to receive processed data from the cloud-based infrastructure, and configured to visualize data to a caregiver. Based on the visualized data, the caregiver can access interesting information and e.g. determine whether a practitioner should be consulted. The caregiver portal may e.g. be a web portal accessible via an internet browser, or an app portal accessible via an application or executable program on a user equipment device

The invention further relates to one or more methods. Although the method(s) can be performed with the system(s) or device(s) according to the invention; neither the system(s)/device(s), nor the method(s) are limited thereto. Features explained herein with reference to the system(s)/device(s) have the same meaning with respect to the method(s) unless explicitly defined otherwise. Features explained with reference to the system(s)/device(s)can be applied mutatis mutandis to the method(s) to achieve the similar advantages, and vice versa.

One or more objects of the invention can be achieved with a method for obtaining a photoplethysmography signal, e.g. for controlling a system for determining a biometric of a patient, wherein the system is a system according to any of the embodiments described herein, wherein the method includes individually controlling: the first intensity and first gain value such that the amplified first PPG-signal is within first signal range; and the second intensity and second gain value such that the amplified second PPG-signal is within second signal range.

One or more objects of the invention can be achieved with a method for controlling a system for obtaining a photoplethysmography signal, e.g. for controlling a system for determining a biometric of a patient, wherein the method comprises the following steps:
- emitting first radiation at a first wavelength onto tissue of the patient with a first intensity, e.g. with a first LED source;
- emitting second radiation at a second wavelength onto the tissue of the patient with a second intensity, e.g. with a second LED source;
- receiving at least a part of the first radiation, generating a first PPG-signal based on the first radiation, and amplifying the first PPG-signal with a first gain value into an amplified first PPG-signal; and
- receiving at least a part of the second radiation, generating a second PPG-signal based on the second radiation, and amplifying the second PPG-signal with a second gain value into an amplified second PPG-signal;
wherein the method includes individually controlling:
- the first intensity and first gain value such that the amplified first PPG-signal is within first signal range; and
- the second intensity and second gain value such that the amplified second PPG-signal is within second signal range.

Optionally, the system is a system according to any of the embodiments described herein.

In embodiments, the method comprises a step of determining at least one biometric of the patient based on both the amplified first PPG-signal and the amplified second PPG-signal.

In embodiments, the method comprises controlling the first intensity to be different from the second intensity and/or the first gain value to be different from the second gain value.

In embodiments, the method comprises individually controlling: the first intensity within a first operational intensity range and first gain value within first operational gain range; and the second intensity within a second operational intensity range and second gain value within second operational gain range. Optionally, the first operational intensity range is different from the second operational intensity range, and/or the first operational gain range is different from the second operational gain range.

In embodiments, the method comprises a calibration of the system, wherein the method comprises the following steps for calibrating the first LED source:
- start emitting the first radiation at a starting intensity which is an upper limit of the first operational intensity range,
- amplify the first PPG-signal with a starting gain value which is an upper limit of the first operational gain range, and
- assess the amplified first PPG-signal, and if the amplified first PPG-signal exceeds the first signal range:
   - lower the first intensity until
      - the amplified first PPG-signal is in the first signal range, or
      - the first intensity reaches a lower limit of the first operational intensity range, and in that case:
         a. lower the first gain value;
         b. increase the first intensity to the upper limit of the first operational intensity range;
- repeat the previous step until the amplified first PPG-signal is within the first signal range.

In embodiments, the method comprises a calibration of the system, wherein the method comprises the following steps for calibrating the second LED source:
- start emitting the second radiation at a starting intensity which is an upper limit of the second operational intensity range,
- amplify the second PPG-signal with a starting gain value which is an upper limit of the second operational gain range, and
- assess the amplified second PPG-signal, and if the amplified second PPG-signal exceeds the second signal range:
   - lower the second intensity until
      - the amplified second PPG-signal is in the second signal range, or
      - the second intensity reaches a lower limit of second first operational intensity range, and in that case:
         a. lower the second gain value;
         b. increase the second intensity to the upper limit of the second operational intensity range;
- repeat the previous step until the amplified second PPG-signal is within second first signal range.

In embodiments, the method further includes doing a recalibration during use, e.g. periodically, wherein the recalibration can e.g. include: assess the amplified first PPG-signal, and if the amplified first PPG-signal is not within the first signal range, control the first intensity and/or the first gain value to be decreased or increased; and/or assess the amplified second PPG-signal, and if the amplified second PPG-signal is not within the second signal range, control the second intensity and/or the second gain value to be decreased or increased.

In embodiments, the method further comprises emitting third radiation at a third wavelength onto tissue of the patient with a third intensity, e.g. with a third LED source.

In embodiments, the method further comprises determining a skin pattern of the tissue of the patient based on calibration data, wherein said calibration data e.g. comprises one or more of the first intensity, the first gain value, the second intensity, and the second gain value, and when present the third intensity and the third gain value. Optionally the method further comprises determining a biometric of the patient, taking into account the skin pattern.

The invention further relates to non-transitory computer-readable instructions configured to, when executed, cause a control unit of a system for obtaining a photoplethysmography signal, e.g. for determining a biometric of a patient, according to any of the embodiments described herein, to perform one or more steps of a method according to any of the embodiments described herein. In particular, the computer-readable instructions may cause a control unit of the system to perform one or more steps and/or control one or more further components to perform one or more steps.

Exemplary embodiments of the invention are described using the figures. It is to be understood that these figures merely serve as example of how the invention can be implemented and are in no way intended to be construed as limiting for the scope of the invention and the claims. It will be understood that in the schematic representations only components relative for the explanation herein are illustrated and/or indicated with reference numerals. The systems and devices may comprise further components that are not explicitly shown but that are known to the skilled person, e.g. for putting the shown components in function in practice as explained herein.

Like features are indicated by like reference numerals along the figures. In the figures:
Fig 1a-1b: schematically illustrate a wearable device;
Fig. 1c: schematically illustrates a patient wearing the wearable device;
Fig. 2: schematically illustrates a system for obtaining a photoplethysmography; signal;
Fig. 3: schematically illustrates a starting calibration and a recalibration;
Fig. 4: schematically illustrates how the system can be used with a cloud-based infrastructure.

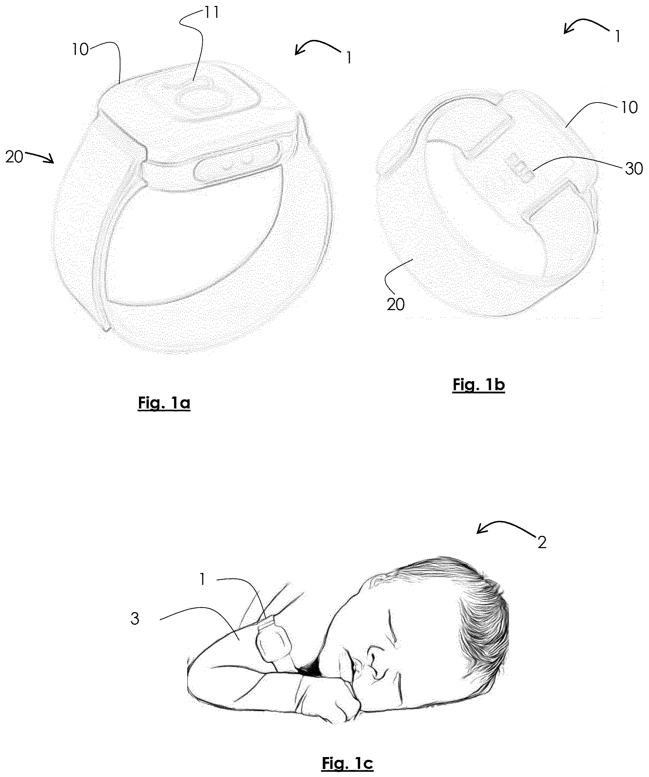

Fig. 1a and fig. 1b schematically illustrate a wearable device 1, and fig; 1c schematically illustrates a patient 2 wearing the wearable device 1. The wearable device 1 comprises a housing 10 and a band 20. The band 20 allows to attach the housing to the patient 2, in this case on the upper arm 3. The patient 2 in this case is a young child.

Fig. 1b illustrates that the wearable device has a PPG-system 30 on the bottom of the housing 10. The PPG-system 30 comprises a plurality of LED sources and photoreceptors. The photoreceptors receive radiation emitted by the LED sources and reflected by the tissue of the patient in the upper arm 3. Based on the reflected radiation, one or more biometrics can be determined.

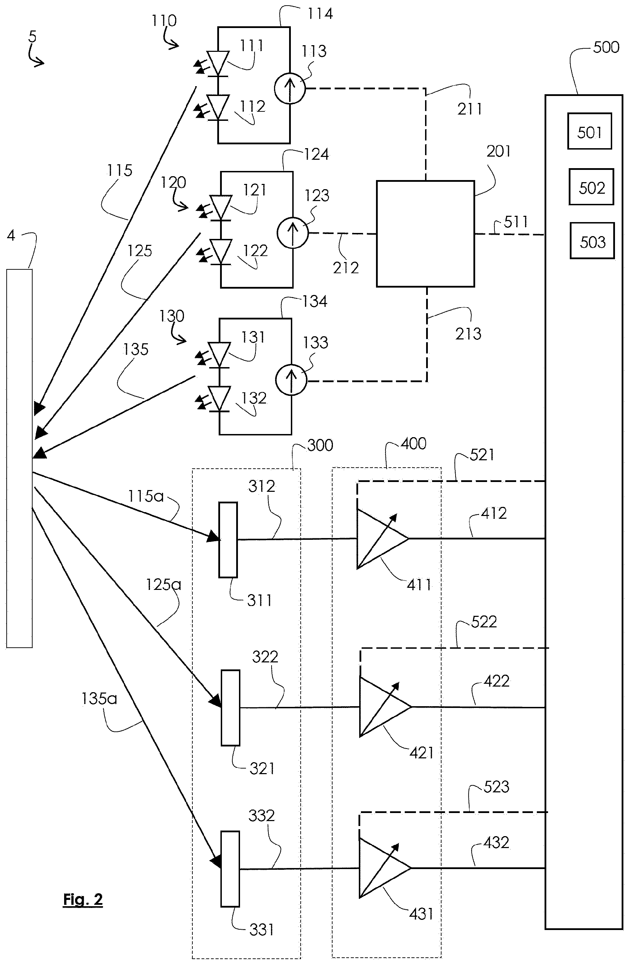

Fig. 2 schematically illustrates a system 5 for determining a biometric, that can e.g. be applied to the wearable device 1 shown in fig. 1a-1c. It is noted however, that the systems and method explained in fig. 2 and generally in this text, can also be used in other devices, e.g. for older patients, and/or non-wearable devices, and/or for devices that analyse radiation that has travelled through tissue rather than being reflected by tissue.

The system 5 shown in fig. 2 comprises a first LED source 110, a second LED source 120, and a third LED source 130. The first LED source 110 is configured to emit first radiation 115 at a first wavelength, which can e.g. be in the red-light spectrum. The second LED source 120 is configured to emit second radiation 125 at a second wavelength, which can e.g. be in the infrared-light spectrum. The third LED source 130 is configured to emit third radiation 135 at a first wavelength, which can e.g. be in the green-light spectrum.

In the shown example, each of the LED sources 110, 120, 130 comprise two LEDs 111,112; 121,122; 131,132. It will be understood however, that this is only a schematical representation, and the exact number of LEDs per LED source 110, 120, 130 may differ and may depend on the device in which the system is used. Moreover, it is possible that one of the LED sources 110, 120, 130 comprises a different number of LEDs than another of the LED sources 110, 120, 130.

The LEDs 111,112; 121,122; 131,132 will emit the respective radiation 115, 125, 135 when sufficient current flows through the respective LEDs 111,112; 121,122; 131,132. Each LED source 110, 120, 130 comprises, therefore, a current source 113, 123, 133. The intensity of the emitted radiation 115, 125, 135 depends on the amount of current flowing through the respective LEDs 111,112; 121,122; 131,132. This is schematically illustrated as a first intensity 114 for the first radiation 115, a second intensity 124 for the second radiation 125, and a third intensity 134 for the third radiation 135.

The first, second, and third radiation 115, 125, 135 are emitted onto tissue 4 of the patient. The tissue 4 can e.g. be part of an upper arm, a lower arm, a wrist, a leg, a hand, a foot, a finger, an ear. The tissue 4 normally comprises at least the skin with which the radiation 115, 125, 135 comes into contact. The tissue 4 includes, besides the skin, also blood vessels with blood. The blood flowing through the blood vessel varies, e.g. in flow, pressure, and composition, depending on various biometrics of the patient. The variation of these aspects of the blood are represented in how much of the radiation 115, 125, 135 is reflected, absorbed, and completely travels through the tissue 4. Based on this, one or more biometrics of the patient can be determined. The tissue 4 may further also comprise e.g. include membranes, muscles, and tendons.

The first 115, second 125, and third radiation 135 are emitted sequentially. For example, first the first radiation 115 is emitted, thereafter the second radiation 125 is emitted, and finally the third radiation 135 is emitted. This is then repeated. When less or more LED sources 110, 120, 130 are present, this can be adapted accordingly.

Fig. 2 illustrates reflected first radiation 115a which is a part of the first radiation 115 that is reflected by the tissue 4. Similarly, reflected second radiation 125a and reflected third radiation 135a are illustrated. Although in the shown embodiment the reflected radiations 115a, 125a, 135a are used for determining the biometrics, the principles explained herein can also be applied to systems wherein radiations travelled through the tissue 4 is used for determining the biometrics.

A receptor system 300 receives a part of each of the first, second, and third radiation 115, 125, 135, wherein in this example said parts are the reflected first, second, and third radiation 115a, 125a, 135a. In the shown example the receptor system 300 comprises a first receptor 311 which receives the reflected first radiation 115a; a second receptor 321 which receives the reflected second radiation 125a; and a third receptor 331 which receives the reflected third radiation 135a.

It is noted that fig. 2 schematically illustrates each receptor 311, 321, 331 receiving one of the reflected first, second, and third radiation 115a, 125a, 135a. In practice, however, it is possible that the physical arrangement of the LED sources 110, 120, 130 and the receptor 311, 321, 331 entails that two or more (or each) of the receptors 311, 321, 331, receives two or more (or each) of the reflected radiations 115a, 125a, 135a. The first, second, and third receptor 311, 321, 331 can e.g. be photoreceptors configured to receive radiation at the respective wavelengths, and filter out radiation at other wavelengths.

In other embodiments, it is also possible that the receptor system 300 comprises one or more photoreceptors for receiving two or more of the reflected first, second, and third radiation 115a, 125a, 135a. In that case said photoreceptor can e.g. be a broadband photoreceptor.

The receptor system 300 is configured to generate a first PPG-signal 312 based on the reflected first radiation 115a. The receptor system 300 is configured to generate a second PPG-signal 322 based on the reflected second radiation 125a. The receptor system 300 is configured to generate a third PPG-signal 332 based on the reflected third radiation 135a. The respective PPG-signals 312, 322, 332 reflect how much of the respective reflected radiation 115a, 125a, 135a is received by the receptor system 300. The PPG-signals 312, 322, 332 thus contain information that is related to one or more biometrics of the patient.

The system 5 further comprises an amplifier system 400. The amplifier system 400 comprises a first amplifier 411 which amplifies the first PPG-signal 312 with a first gain value into an amplified first PPG-signal 412. The amplifier system 400 comprises a second amplifier 421 which amplifies the second PPG-signal 322 with a second gain value into an amplified second PPG-signal 422. The amplifier system 400 comprises a third amplifier 431 which amplifies the third PPG-signal 332 with a third gain value into an amplified third PPG-signal 432. It will be understood, however, that although the shown example shows three amplifiers 411, 421, 431 for three PPG-signals, different implementations may be possible.

Amplifying the PPG-signals 312, 322, 332 into the respective amplified PPG-signals 412, 422, 432 may be advantageous for increasing the quality of the signal, and thereby improving the determination of the biometrics based on said signals.

The system 5 further comprises a control unit 500. The control unit 500 receives the amplified PPG-signals 412, 422, 432. The control unit 500 comprises a processing unit 501, e.g. for processing those amplified PPG-signals 412, 422, 432, and determining one or more biometrics of the patient. It is also possible, however, that the biometrics are determined by a further control unit (not shown). The further control unit may e.g. receive the amplified PPG-signals 412, 422, 432 as raw data, or the control unit 500 may perform a preprocessing on the amplified PPG-signals 412, 422, 433 and transmit processed data to the further control unit.

The control unit 500 can e.g. be configured to determine an SpO2 level of the patient, e.g. based on two of the amplified PPG-signals 412, 422, 433. The control unit 500 can also be configured to determine a pulse rate of the patient or a respiratory rate of the patient, e.g. based on one or more of the amplified PPG-signals 412, 422, 433.

The control unit 500 comprises a memory 502. The memory 502 can e.g. store computer-readable instructions, based on which the control unit 500 can process the amplified PPG-signals 412, 422, 423. The memory 502 can e.g. store raw data and/or processed data based on the amplified PPG-signals 412, 422, 423. The control unit 500 comprises a wireless communication unit 503 which may e.g. be used for establishing a wireless connection. The wireless connection may use any suitable communication technique, e.g. including Wi-Fi, 3G, 4G, 5G, Bluetooth.

In order to allow accurate determination of the biometrics, the amplified PPG-signals 412, 422, 432 must be in a useful range. That is, these signals should not be too small to extract useful information, while at the same time they should not be so large that they cause saturation. A signal range can be defined in which a respective amplified PPG-signal 412, 422, 432 should be. The quality of the signals depends on several factors. A first factor is the intensities 114, 124, 134 with which the radiation 115, 125, 135 is emitted. A second factor is how much of the radiation 115, 125, 135 is reflected as reflected radiation 115a, 125a, 135a. Yet another factor is the applied gain values by the amplifier system 400.

How much of the radiation 115, 125, 135 is reflected as reflected radiation 115a, 125a, 135a, in turn also depends on various factors that can be patient specific. Characteristics of the tissue 4 such as skin tone, skin thickness, tattoos, scars, skin temperature, have been found to play a role. Interestingly, the inventors have found that the effect the tissue 4 has on the (reflection of the) radiation 115, 125, 135, also depends on the wavelength of the radiation.

It may therefore be advantageous to individually control: the first intensity 114 and first gain value such that the amplified first PPG-signal 412 is within a first signal range; the second intensity 124 and second gain value such that the amplified second PPG-signal 422 is within a second signal range; and the third intensity 134 and third gain value such that the amplified third PPG-signal 432 is within a third signal range.

The control unit 500 is in particular configured to control the intensifier system 201 with an intensity control signal 511. Based on the intensity control signal 511, the intensifier system 201 can generate a first intensity signal 211 for the first LED source 110; a second intensity signal 212 for the second LED source 120; and a third intensity signal 213 for the third LED source 130. In the shown example, the respective intensity signals 211, 212, 213 control the respective current sources 113, 123, 133, such that the amount of current flowing through the LEDs 111,112; 121,122; 131,132 can be controlled. The intensity of the radiation 115, 125, 135 is dependent on the current, and can thus be controlled in this manner.

The control unit 500 is further configured to control the first amplifier 411 with a first amplifier control signal 521; the second amplifier 421 with a second amplifier control signal 522; and the third amplifier 431 with a third amplifier control signal 531. The respective amplifier control signals 521, 522, 523 can control the respective gain values applied by the respective amplifier 411, 421, 431.

The control unit 500 may in particular control two or more of the first intensity 114, the second intensity 124, and the third intensity 134 to be mutually different. The control unit 500 may also control two or more of the first gain value, the second gain value, and the third gain value to be mutually different. It will regularly occur that the optimal configuration for the particular wavelengths of the radiation 115, 125, 135 and the tissue of the patient 4, entails said intensities and gain values are mutually different (although it is noted that in some configuration settings, one or more of the intensities or gain values can also be equal). Since each of said intensities and gain values is individually controlled, this is possible.

The control unit 500 may control the different parameters to be within respective operational ranges. That is, the first intensity 114 may be controlled to be within a first operational intensity range; the second intensity 124 to be within a second operational intensity range; and the third intensity 134 to be within a third operational intensity range. The first gain value may be controlled to be within a first operational gain range; the second gain value to be within a second operational gain range; and the third gain value to be within a third operational gain range.

For any of the wavelengths of the radiation 115, 125, 135 the absorption and reflection may depend on the tissue 4 of the particular patient. Nevertheless, it may still be possible to define an operation range for the respective intensity and gain value for which a satisfactory amplified PPG-signal can be obtained (at least for most patients). By limiting the intensity and gain value to said respective operational ranges, the optimal amplified PPG-signal can be obtained faster and in a more efficient way in view of power consumption.

The reflection and absorption depend also on the wavelength of the radiation. It may therefore be possible to define specific operational ranges for the intensity and gain value in function of the wavelength. Therefore, the respective operational ranges may be different for each of the wavelengths. That is, two or more of the first operational intensity range, the second operational intensity range, and the third operational intensity range may be mutually different. Also two or more of the first operational gain range, the second operational gain range, and the third operational gain range may be mutually different.

Even when specific operation ranges are defined for the intensity and gain value for each wavelength, the respective amplified PPG-signal will in most cases not be within the respective signal ranges for all possible combinations of possible intensity and gain value within said operational ranges. The control unit 500 may therefore be configured to perform a calibration of the system 5.

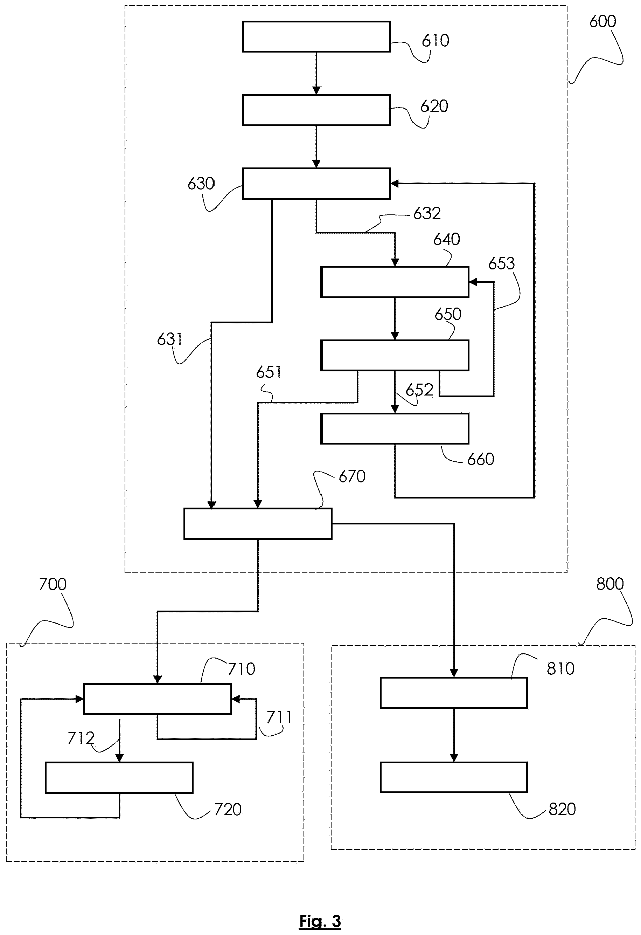

Fig. 3 schematically illustrates the calibration can be performed in some embodiments, which are elaborated on in with reference to fig. 2 and fig. 3. The control unit 500 may be configured to perform the steps shown in fig. 3, and/or control one or more components of the system 5 to perform the steps. The calibration is explained with reference to the first LED source 110; however, it will be understood that the same principles can be applied to the second LED source 120 and the third LED source 130, mutatis mutandis.

When the system 5 is started, a starting calibration 600 can be performed. The starting calibration 600 starts with a step 610 in which the first LED source 110 and the intensifier system 201 are controlled to start emitting the first radiation 115 at a starting intensity 114 which is an upper limit of the first operational intensity range. Although not explicitly shown in fig. 3, it will be understood that reflected first radiation 115a is received and converted into a first PPG-signal312 by the first receptor311.The starting calibration 600 then includes a step 620 wherein the amplifier system 400 is controlled to amplify the first PPG-signal 312 with a starting gain value which is an upper limit of the first operational gain range. The amplified first PPG-signal 412 is then assessed in step 630. In case the amplified first PPG-signal 412 is within the first signal range, the starting calibration 600 for the first LED source 110 can be concluded in step 670 (thus following arrow 631 in fig. 3).

If the amplified first PGG-signal 412 is not within the first signal range, the starting calibration 600 proceeds to step 640 (thus following arrow 632 in fig. 3). In step 640, the intensifier system 201 lowers the first intensity 114. The amplified first PPG-signal 412 is then again assessed in step 650. In case the amplified first PPG-signal 412 is within the first signal range, the starting calibration 600 for the first LED source 110 can be concluded in step 670 (thus following arrow 651 in fig. 3). In case the amplified first PPG-signal 412 is not within the first signal range and the first intensity 114 is not yet at a lower limit of the first operational intensity range, the starting calibration 600 follow arrow 653 back to step 640 to further lower the first intensity 114. Once the lower limit of the first operational intensity range is reached without being within the first signal range, the starting calibration 600 follows arrow 652 to step 660. In step 660, the first gain value is lowered, and the first intensity is set back to the upper limit. The starting calibration 600 then returns to step 630. If the amplified first PGG-signal 412 is still not within the first signal range, the starting calibration 600 follows the steps 640-660 again.

The starting calibration 600 thus starts with the intensity and gain value at the upper limits of their respective operational ranges. This can be advantageous because the amplified PPG-signal is preferably as large as possible while remaining in the respective signal range. If the amplified PPG-signal is not within the signal range, first the intensity is lowered until the lower limit of the operational intensity range. Then, the gain value range is lowered, and the intensity is arranged back at upper limit and lowered if needed. By applying this process, all combinations of intensity and gain value can be included, if needed. It will be understood, that a similar starting calibration can be applied to the second LED source 120 and the third LED source 133. It will also be understood that in other embodiments it can be possible to start the intensity and/or gain value at the lower limits of their operational ranges.

Fig. 3 further illustrates that optionally a recalibration 700 can be applied. The recalibration 700 can be advantageous, because the reflection and absorption of the radiation 115, 125, 135 by the tissue 4 can change during use. This may e.g. because the temperature of the tissue 4 slightly changes, e.g. because of the radiation 115, 125, 135. It can also occur that that the tissue 4 and the system 5 are slightly moved relative to each other, causing the radiation 115, 125, 135 to come into contact with the tissue 4 at another location (with other characteristics).

The recalibration 700 includes a step 710 wherein the amplified first PPG-signal 412 is assessed. If the first PPG-signal 412 is still within the first signal range, no changes have to be made and the recalibration can return to step 710 (thus following arrow 711 in fig. 3) to reassess the first PPG-signal 412 after waiting a sufficient period of time. If, on the other hand, the amplified first PPG-signal 412 is not within the first signal range, the recalibration follows arrow 712 to step 720. In step 720, the control unit 500 decreases or increases the first intensity and/or the first gain value such that the amplified first PPG-signal 412 returns to first signal range. The control unit 500 may be configured to first adapt the first intensity. Depending on whether the amplified first PPG-signal is below or above the first signal range, the first intensity is increased or decreased, respectively. When an upper or lower limit of the first intensity range is reached, the gain value is changed. It will be understood that a similar recalibration can be applied for the second LED source 120 and the third LED source 130, mutatis mutandis.

The recalibration 700 is performed periodically, for example every 100 ms. It is advantageous that the time period in between two recalibrations is not too long, to avoid the amplified PPG-signal being out of the signal range for too long. At the same time, the system 5 may require some stabilisation time before the changes done during a recalibration stabilize. The stabilisation time may depend on the hardware.

Although fig. 3 illustrates three LED sources 110, 120, 130, it will be understood that the system 5 may comprise less or more LED sources, while applying the principles explained herein.

Fig. 3 further illustrates a method 800. In the method 800, first a skin pattern is determined by the control unit 500 in step 810. The skin pattern is determined based on calibration data, which includes the data obtained in step 670. The calibration data includes the first intensity and the first gain value, and similarly the intensities and gain values for the other present LED sources and/or wavelengths. The calibration is dependent on the skin pattern of the patient, which represents a combination of characteristics of the tissue of the patient. For example, seven skin patterns can be defined, each representing a cluster of possible calibration data.

In step 820, a biometric is determined by the control unit 500. The control unit 500 takes into account the skin pattern determined in step 810, which allows to determine the biometric more accurately. For example, the control unit 500 can be configured to determine an SpO2 level as biometric in step 820, wherein the control unit is configured to determine a ratio of ratios, and convert the ratio of ratios to an SpO2 level using a transfer function. The control unit 500 can be configured to determine or select the transfer function based on the skin pattern.

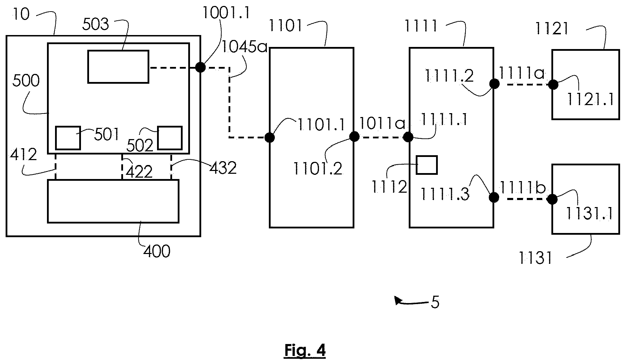

Fig. 4 schematically illustrates how the system 5 can be used with a cloud-based infrastructure 1111. A local user equipment device 1101 is illustrated. The local user equipment device 1101 can e.g. be a tablet or smartphone, and is arranged in the vicinity of the wearable device. Optionally the local user equipment device 1101 is pre-programmed by the operator of the system 5. The local user equipment device 1101 is configured to receive raw/and or processed data via communication signal 1045a from the control unit 500, e.g. via communication terminals 1001.1; 1101.1. The local user equipment device 1101 may be configured to process raw/and or processed data, and/or to visualize the data received from the control unit 500. The local user equipment device 1101 may e.g. comprise a processing unit, memory, and/or screen.

The system 5 further comprises a cloud-based infrastructure 1111 configured to receive a cloud communication signal 1101a from the local user equipment device 1101, e.g. via communication terminals 1101.2, 1111.1. The cloud communication signal 1101a may e.g. comprise raw and/or processed data. The cloud-based infrastructure 1111 comprises a server having a processing unit 1112 for further processing said raw and/or processed data. The cloud-based infrastructure 1111 may e.g. be configured to communicate the cloud communication signal 1101a with the local user equipment device 1101a via an internet communication, e.g. WI-FI, 3G, 4G, or 5G.

The system 5 further comprises a practitioner portal 1121, configured to receive processed data from the cloud-based infrastructure 1111. This is achieved by means of a practitioner communication signal 1111a communicated via communication terminals 1111.2, 1121.1. The practitioner portal 1121 is configured to visualize data to a practitioner. Based on the visualized data, the practitioner can determine the health of the patient and propose the suitable medical treatment when required. The practitioner portal 1121 may e.g. be a web portal accessible via an internet browser, or an app portal accessible via an application or executable program on a user equipment device.

The system 5 further comprising a caregiver portal 1131, configured to receive processed data from the cloud-based infrastructure 1111. This is achieved by means of a caregiver communication signal 1111b communicated via communication terminals 1111.3, 1131. 1. The caregiver portal 1131 is configured to visualize data to a caregiver. Based on the visualized data, the caregiver can access interesting information and e.g. determine whether a practitioner should be consulted. The caregiver portal may e.g. be a web portal accessible via an internet browser, or an app portal accessible via an application or executable program on a user equipment device.

As required, detailed embodiments of the present invention are described herein; however, it is to be understood that the disclosed embodiments are merely examples of the invention, which may be embodied in various ways. Therefore, specific structural and functional details disclosed herein are not to be construed as limiting, but merely as a basis for the claims and as a representative basis for teaching those skilled in the art to practice the present invention in various ways in virtually any suitable detailed structure. Not all of the objectives described need be achieved with particular embodiments.

Furthermore, the terms and expressions used herein are not intended to limit the invention, but to provide an understandable description of the invention. The words "a", "an", or "one" used herein mean one or more than one, unless otherwise indicated. The terms "a multiple of", "a plurality" or "several" mean two or more than two. The words "comprise", "include", "contain" and "have" have an open meaning and do not exclude the presence of additional elements. Reference numerals in the claims should not be construed as limiting the invention.

The mere fact that certain technical features are described in different dependent claims still allows the possibility that a combination of these technical measures can be used advantageously.

A single processor or other unit can perform the functions of various components mentioned in the description and claims, e.g. of processing units or control units, or the functionality of a single processing unit or control unit described herein can in practice be distributed over multiple components, optionally physically separated of each other. Any communication between components can be wired or wireless by known methods.

The actions performed by the control unit can be implemented as a program, for example computer program, software application, or the like. The program can be executed using computer readable instructions. The program may include a subroutine, a function, a procedure, an object method, an object implementation, an executable application, a source code, an object code, a shared library / dynamic load library and / or other set of instructions designed for execution on a computer system.

A computer program or computer-readable instructions can be stored and / or distributed on a suitable medium, such as an optical storage medium or a solid-state medium supplied with or as part of other hardware, but can also be distributed in other forms, such as via internet or other wired or wireless telecommunication systems.

## Claims

1. A system for obtaining a photoplethysmography signal, comprising:
• a first LED source configured to emit first radiation at a first wavelength onto tissue of the patient;
• a second LED source configured to emit second radiation at a second wavelength onto the tissue of the patient;
• an intensifier system configured to control:
• the first LED source to emit the first radiation with a first intensity, and
• the second LED source to emit the second radiation a second intensity;
• a receptor system configured to:
• receive at least a part of the first radiation and generate a first PPG-signal based on the first radiation; and
• receive at least a part of the second radiation and generate a second PPG-signal based on the second radiation;
• an amplifier system configured to:
• receive the first PPG-signal from the receptor system, and amplify the first PPG-signal with a first gain value into an amplified first PPG-signal; and
• receive the second PPG-signal from the receptor system, amplify the second PPG-signal with a second gain value into an amplified second PPG-signal;
• a control unit configured to individually control:
• the first intensity and first gain value such that the amplified first PPG-signal is within a first signal range;
• the second intensity and second gain value such that the amplified second PPG-signal is within a second signal range.

2. The system according to claim 1, wherein the control unit is further configured to determine at least one biometric of the patient based on both the amplified first PPG-signal and the amplified second PPG-signal.

3. The system according to any of the preceding claims, wherein the control unit is further configured to control the first intensity to be different from the second intensity and/or the first gain value to be different from the second gain value.

4. The system according to any of the preceding claims, wherein the control unit is configured to individually control:
• the first intensity within a first operational intensity range and first gain value within first operational gain range; and
• the second intensity within a second operational intensity range and second gain value within second operational gain range.

5. The system according to claim 4, wherein the first operational intensity range is different from the second operational intensity range, and/or wherein the first operational gain range is different from the second operational gain range.

6. The system according to any of the preceding claims 4-5, wherein the control unit is configured to perform a calibration of the system, wherein during the calibration the control unit is configured to, for calibrating the first LED source:
• control the first LED source and the intensifier system to start emitting the first radiation at a starting intensity which is an upper limit of the first operational intensity range,
• control the amplifier system to amplify the first PPG-signal with a starting gain value which is an upper limit of the first operational gain range, and
• assess the amplified first PPG-signal, and if the amplified first PPG-signal exceeds the first signal range:
• control the intensifier system to lower the first intensity until
• the amplified first PPG-signal is in the first signal range, or
• the first intensity reaches a lower limit of the first operational intensity range, and in that case:
a. control the amplifier system to lower the first gain value;
b. control the intensifier system increase the first intensity to the upper limit of the first operational intensity range;
• repeat the previous step until the amplified first PPG-signal is within the first signal range.

7. The system according to claim 6, wherein the control unit is further configured to, for calibrating the second LED source:
• control the second LED source and the intensifier system to start emitting the second radiation at a starting intensity which is an upper limit of the second operational intensity range,
• control the amplifier system to amplify the second PPG-signal with a starting gain value which is an upper limit of the second operational gain range, and
• assess the amplified second PPG-signal, and if the amplified second PPG-signal exceeds the second signal range:
• control the intensifier system to lower the second intensity until
• the amplified PPG-signal is in the second signal range, or
• the second intensity reaches a lower limit of the second operational intensity range, and in that case:
a. control the amplifier system to lower the second gain value;
b. control the intensifier system increase the second intensity to the upper limit of the second operational intensity range;
• repeat the previous step until the amplified second PPG-signal is within the second signal range.

8. The system according to claim 6 or claim 7, wherein the control unit is further configured periodically perform a recalibration during use, e.g. every 100 ms, wherein during the recalibration the control unit is configured to:
• assess the amplified first PPG-signal, and if the amplified first PPG-signal is not within the first signal range, control the first intensity and/or the first gain value to be decreased or increased; and/or
• assess the amplified second PPG-signal, and if the amplified second PPG-signal is not within the second signal range, control the second intensity and/or the second gain value to be decreased or increased.

9. The system according to any of the preceding claims, wherein the control unit is configured to
• determine a skin pattern of the tissue of the patient based on calibration data, wherein said calibration data comprises one or more of the first intensity, the first gain value, the second intensity, and the second gain value, and when present the third intensity and the third gain value;
• determine a biometric of the patient, taking into account the skin pattern.

10. System for determining at least one biometric of a patient, comprising a wearable device comprising a system according to one or more of the preceding claims, and a local user equipment device, wherein the user equipment device is configured to wireless communicate with the wearable device for receiving raw and/or processed data based on measurement of the at least one sensor of the wearable device.

11. System according to the preceding claim, further comprising a cloud-based infrastructure configured to receive raw and/or processed data from the local user equipment device, and comprising at least one server having a processing unit for further processing said raw and/or processed data.

12. System according to the preceding claims 10 or 11, further comprising a practitioner portal, configured to receive processed data from the cloud-based infrastructure, and configured to visualize data to a practitioner.

13. System according to the preceding claim, further comprising caregiver portal, configured to receive processed data from the cloud-based infrastructure, and configured to visualize data to a caregiver..

14. Method for controlling a system for determining a biometric of a patient, wherein the method comprises the following steps:
• emitting first radiation at a first wavelength onto tissue of the patient with a first intensity;
• emitting second radiation at a second wavelength onto the tissue of the patient with a second intensity;
• receiving at least a part of the first radiation, generating a first PPG-signal based on the first radiation, and amplifying the first PPG-signal with a first gain value into an amplified first PPG-signal, and amplifying the second PPG-signal with a second gain value into an amplified second PPG-signal;
wherein the method includes individually controlling:
• the first intensity and first gain value such that the amplified first PPG-signal is within first signal range; and
• the second intensity and second gain value such that the amplified second PPG-signal is within second signal range.

15. Non-transitory computer-readable instructions configured to, when executed, cause a control unit of a system for determining a biometric off a patient according to any of the preceding claims 1-13 to perform a method according to claim 14.
